Europäisches Patentamt

(19) European Patent Office

Office européen des brevets

(11) Publication number: **0 011 739**
**B1**

(12) **EUROPEAN PATENT SPECIFICATION**

(45) Date of publication of patent specification: **25.05.83**

(51) Int. Cl.³: **C 07 G 7/00, A 61 K 35/16**

(21) Application number: **79104346.6**

(22) Date of filing: **06.11.79**

(54) **Process for obtaining blood coagulation factor XIII derived from human placenta.**

(30) Priority: **07.11.78 JP 136996/78**

(43) Date of publication of application:
**11.06.80 Bulletin 80/12**

(45) Publication of the grant of the patent:
**25.05.83 Bulletin 83/21**

(84) Designated Contracting States:
**CH DE FR GB SE**

(56) References cited:
**US - A - 3 904 751**
**US - A - 3 931 399**

**Chemical Abstracts vol. 89, no. 12, 18
September 1978
Columbus, Ohio, USA
T. FUKUSHIMA et al. "Isolation of blood
coagulation factor XIII from human placentas"
page 307, column 1, abstract no. 94997t**

(73) Proprietor: **The Green Cross Corporation
1-47, Chuo-1-chome
Joto-ku, Osaka (JP)**

(72) Inventor: **Fukushima, Tunekazu
18-2, Shioyadai-3-chome
Tarumi-ku, Kobe (JP)**
Inventor: **Funakoshi, Satoshi
16-5, Aoyama-1-chome
Katano-shi (JP)**

(74) Representative: **Vossius Vossius Tauchner
Heunemann Rauh
Siebertstrasse 4 P.O. Box 86 07 67
D-8000 München 86 (DE)**

Process for Obtaining Blood Coagulation Factor XIII derived from Human Placenta

The present invention relates to a process for obtaining blood coagulation Factor XIII (fibrin stabilizing factor, hereinafter referred to as Factor XIII) derived from human placenta.

Much attention has recently been drawn to the clinical applications of Factor XIII, which studies of the thrombus forming mechanism have shown to contribute in the field of wound healing to the formation of insoluble fibrin in the fibrinogen-fibrin system.

The abnormal symptoms of protracted bleeding and retarded healing of wounds observed in cases of a congenital deficiency of Factor XIII are known to be caused by incomplete formation of cross-linking between $\gamma$-chains of the fibrin which forms a substrate of Factor XIII. Furthermore, not only the cross-linking between $\gamma$-chains of fibrin, but also the biological significance of the cross-linking between $\alpha$-chains have attracted much interest. The cross-linking between $\gamma$-chains of fibrin regulates its chemical stability, whereas that between $\alpha$-chains regulates the physical and mechanical strength of fibrin. It has, moreover, been found that a cold-insoluble globulin which had hitherto been regarded merely as a contaminant always present when fibrinogen and the antihemophilic factor were purified evidently plays a biologically important role as a substrate of Factor XIII like fibrin. In other words, the present view is that when a wound begins to heal the primary wound healing phase seems to be dependent on Factor XIII, so that the cross-linking between $\alpha$-chains of fibrin develops among not only fibrin, but also among cold-insoluble globulins. The following publications are mentioned by way of reference:

(1) Pisano, J. J., "Ann. N.Y. Acad. Sci." *202*, 98 (1972);

(2) McDonagh R. P., "The 16th International Congr. Hematol., Kyoto" 1976;

(3) Mosher, D. F., "J. Biol. Chem." *250*, 6614 (1975).

From the foregoing it is evident that with regard to clinical application the Factor XIII preparation is highly effective for a wide range of purposes, e.g. for remedying both congenital and acquired deficiencies of Factor XIII as well as for promoting general post-operative wound healing.

With regard to the starting materials for preparing Factor XIII, human placenta is superior from the point of view of price and yield and from the practical aspect of the manufacture of ready-made pharmaceutical preparations.

Japanese Patent Application (Kokai; Laid-Open) No. 59018/1978 (see Derwent Abstract 75-3059-018) discloses that various studies have been made in developing a process for producing Factor XIII and gave a solution to the problem of developing such a process connected with the production of ready-made pharmaceutical compositions such as albumin and $\gamma$-globulin. Said Japanese Laid-Open Patent Application found that Factor XIII is concentrated in a crude globulin fraction obtained in the step for producing albumin and $\gamma$-globulin from placenta, which step consists mainly of an ammonium sulfate fractionation method. Based on this finding a process for producing Factor XIII was perfected, the process further including a step for an inactivating treatment of the hepatitis virus, which is a common problem in blood products.

The process of said Japanese Patent Application (Kokai; Laid-Open) No. 59018/1978 is outlined below.

a) Mucoid protein is removed as a precipitate by centrifugation from placenta with a sodium chloride solution;

b) the thus obtained supernatant liquor is brought to a pH of 5 to 7, an ammonium sulfate saturation of 20 to 30% and a temperature of 1° to 15°C, thereby removing as a precipitate tissue protein contained therein;

c) the resulting supernatant is brought to a pH of 6 to 8 and an ammonium sulfate saturation of 45 to 55% to obtain a crude globulin fraction as a precipitate and the resulting supernatant is utilized as a crude albumin fraction;

d) acrinol is added to said crude globulin fraction in an amount of 1—2% (W/V) and left to stand in this state for one night at a pH of 8.0 to 9.0 to separate $\gamma$-globulin and hemoglobin as precipitates;

e) the resulting supernatant is filtered and sodium chloride is added in a concentration of 2—8% (W/V) to the filtrate to remove the remaining acrinol, and after filtering out the precipitate, an absorbent selected from bentonite, active carbon, acid clay, etc. is added to the filtrate to remove the remaining acrinol;

f) the resulting solution is filtered to remove precipitates, ammonium sulfate is added to the solution in a concentration of 40 to 50% saturation and the $\gamma$-globulin fraction is separated as a precipitate and recovered as a supernatant, concentrated Factor XIII fraction.

g) to the concentrated Factor XIII fraction is added a stabilizer selected from the group consisting of neutral amino acids, monosaccharides, and sugar alcohols, either alone or in combination, in a concentration of 10 tc 20% (W/V), and then the solution is subjected to heat treatment for 9 to 11 hours at 55° to 65°C to induce the formation of heat-unstable substances and to inactivate the hepatitis virus;

h) the heat-treated solution is then subjected to chromatographic treatment with an anion exchanger or block electrophoresis to separate heat-unstable substances;

i) finally, the obtained solution is dialyzed, sterilized by filtration and freeze-dried according to known methods.

As is evident from the above, the process disclosed in Japanese Patent Application (Kokai; Laid-Open) No. 59018/1978 utilizes a number of steps to obtain Factor XIII after separating a crude globulin fraction and a crude albumin fraction by the ammonium sulfate fractionation method. Accordingly, contamination of pyrogens in the obtained Factor XIII has often been observed.

Upon making intensive studies on this point it has now been found that by applying an alkylene oxide polymer or copolymer fractionation method to the purification of the crude globulin fraction it is possible to simplify the steps and thereby enhance the prevention of pyrogen contamination, and furthermore to achieve an increase in yield. Moreover, the purity and stability of the product obtained according to the present invention are not different from those of the product of Japanese Patent Application (Kokai; Laid-Open) No. 59018/1978.

According to the present invention there is provided a process for obtaining concentrated blood coagulation Factor XIII derived from human placenta by

1) extracting the placenta with a physiologically saline solution,

2) removing precipitates from the solution by centrifugation,

3) adding ammonium sulfate to the supernatant in a concentration of 20 to 30% (W/V),

4) recovering the supernatant and removing the precipitate therefrom,

5) adding ammonium sulfate to the supernatant in a concentration of 45 to 55% (W/V) and recovering the precipitate formed,

6) dissolving the precipitate in a phosphate buffer solution of a pH of 6 to 9 and containing about 0.005M of EDTA,

7) fractionating the Factor XIII containing solution for further purification,

8) heat-treating the purified Factor XIII containing solution for 9 to 11 hours at 55 to 65°C in the presence of a neutral amino acid, a monosaccharide, a sugar alcohol or a mixture of two or more thereof in a concentration of 10 to 20% (W/V),

9) contacting the resulting solution with a DEAE-cross-linked dextran which had been equilibrated to a pH of 6 to 9 thereby to adsorb blood coagulation Factor XIII,

10) eluting the blood coagulation Factor XIII,

11) subjecting the collected eluate to dialysis, sterilizing filtration and freeze drying, characterized in that the fractionation step (7) comprises the following steps

a) adding an alkylene oxide polymer or copolymer having a molecular weight of 2,000 to 20,000 selected from the group consiting of a polyethylene oxide homopolymer, a polypropylene oxide homopolymer and an ethylene oxide-propylene oxide copolymer in a concentration of 4 to 9% W/V to the solution,

b) removing the precipitate formed and recovering the supernatant,

c) adding the above-mentioned alkylene oxide polymer or copolymer to the supernatant in a concentration of 20 to 30% (W/V) and

d) recovering the precipitate formed and dissolving it in a phosphate buffer of a pH of 6 to 9.

The present invention will be explained in more detail in the following.

The crude globulin fraction derived from human placenta used in the process of the present invention is obtained from a placenta extract solution produced according to known methods such as, for example, disclosed in Japanese Patent Application (Kokai; Laid-Open) No. 59018/1978. In other words foreign mucoid colloidal proteins are removed by centrifugation from the extract solution of finely chopped or minced human placenta using a saline solution. The supernatant is then saturated with ammonium sulfate in a concentration of 20 to 30% at a temperature of 1° to 15°C and at a pH of 5 to 7 to precipitate and remove tissue proteins. The resulting supernatant, which is a crude albumin fraction, is increased in its ammonium sulfate concentration to 45 to 55% solution at a pH of 6 to 8 to precipitate a fraction and then separate the fraction from the supernatant.

The obtained crude globulin fraction is subjected to fractionation with an alkylene oxide polymer or copolymer into a $\gamma$-globulin fraction, a hemoglobulin fraction and a fraction containing a large amount of Factor XIII according to the method of the present invention.

More specifically, the crude globulin fraction obtained as a precipitate in the above step is dissolved in a buffer solution having a pH of 6 to 9, preferably 7, which contains EDTA (ethylenediaminetetraacetic acid salt) as divalent metal chelating agent. To the obtained solution is added, alone or in combination, an alkylene oxide polymer or copolymer (referred to hereinafter as polyol) having a molecular weight of 2,000 to 20,000, such a polyoxyethylene or polyoxypropylene homopolymer or polyoxyethylene-polyoxypropylene copolymer (PLURONIC®, polyol). First of all the polyol is dissolved in the solution in a concentration of 4 to 9% (W/V), preferably 7% (W/V), to form a precipitate ($\gamma$-globulin fraction) which is removed by centrifugation. To the obtained supernatant the polyol is again added to make a final concentration of 20 to 30% (W/V), preferably 25% (W/V). The thus formed precipitate, which is Factor XIII, is collected by separating it from the supernatant (which is a hemoglobin fraction).

As is evident from the above, the difference between the process described in Japanese Patent Application No. 59018/1978 and that of the present invention is as follows: the fractionation steps with (a) acrinol and (b) NaCl, (c) contacting with bentonite and (d) fractionation with ammonium sulfate are replaced by the fractionation steps with an alkylene oxide polymer.

In consequence, the application of two fractionation steps, namely steps 7(a) and (c), gives better results.

The desired concentrated Fraction XIII thus obtained is subjected, according to known methods, to ion-exchange chromatography with DEAE-Sephadex®, dialysis, sterilization by filtration and freeze-drying to obtain a purified fraction which can be used for the preparation of pharmaceutical compositions.

However, it is desirable that said purified fraction be subjected to heat treatment before making pharmaceutical preparations in order to inactivate the hepatitis virus which may exist in the fraction. The inactivation method is the same as that disclosed in Japanese Patent Application (Kokai; Laid-Open) No. 59018/1978. In other words, the inactivation is carried out by heating a buffer solution containing said fraction having a pH of 6 to 9 at a temperature of 55° to 65°C for 9 to 11 hours in the presence of a stabilizer such as a neutral amino acid, monosaccharide or sugar alcohol in a concentration of 10 to 20% (W/V). Heat-unstable substances which are formed during the heat treatment can be removed by means of chromatography with an anion exchanger such as DEAE-Sephadex® or with block electrophoresis after cooling the hot solution. Thereafter the fraction can be used for the preparation of pharmaceutical compositions.

The pharmaceutical compositions prepared from Factor XIII obtained as outlined above contain 250 units in 4 ml, if the active amount of Factor XIII contained in 1 ml of normal fresh serum is fixed as one unit.

The present invention will be illustrated below with examples for the purpose of illustration only; the invention is not limited to the contents of the embodiments shown in the examples.

### Example 1

800 millilitres of an extract obtained by extracting human placenta with normal saline solution (using about 840 placentae weighing about 510 kilograms in all) were separated by centrifugation. The pH of the filtrate was adjusted to 5.0 and the resulting precipitate was separated. To the supernatant solution was added ammonium sulfate at a pH of 6 to a saturation of 25% to form a precipitate which was removed by centrifugation.

When ammonium sulfate was added to the obtained supernatant to a saturation of 70%, a precipitate of a crude globulin fraction was formed. The fraction was collected by centrifugation.

3.5 kilograms of the thus obtained crude globulin fraction were dissolved in 100 litres of a 0.05 Mol phosphate buffer solution having a pH of 7.5 and containing 0.005 Mol of EDTA. To the solution was added 7.0 kg of PLURONIC (a copolymer of polyoxyethylene and polyoxypropylene having a mean molecular weight of 15,000) and dissolved therein. After the solution had stood for one night as it was, the formed precipitate (which can be used for the purification of globulin) was removed by centrifugation. PLURONIC® was again added to the obtained supernatant and increased in concentration to reach a final concentration of 25% (W/V). After letting the solution stand as it was for one night, the resulting precipitates were collected and dissolved in the same buffer solution as mentioned above. The solution was then subjected to heat treatment at 60°C for 10 hours, ion-exchange chromatography using DEAE-Sephadex, dialysis, sterilization by filtration and adjusting to dosage, followed by freeze-drying. 187 grams of protein were obtained as a product containing 2.2 million units of the Factor XIII, if the activity of Factor XIII contained in 1 ml of normal human serum is fixed as one unit.

### Example 2

Now using 4.0 kilograms of polyoxypropylene glycol having a mean molecular weight of 8,000 in place of the PLURONIC® used in Example 1, globulin was removed therefrom, and then polyoxypropylene polymer was again added to the solution and its concentration increased to a final concentration of 20% (W/V). The same purification methods as in Example 1 were repeated, with the exception of the above-mentioned procedure. As a result, 151 grams of protein containing 1.8 million units of concentrated Factor XIII were obtained.

### Example 3

9.0 kg of polyoxyethylene glycol (mean molecular weight: 12,000) were used in place of the PLURONIC® used in Example 1. First of all globulin was removed. Thereafter polyoxyethylene glycol was again added and the concentration thereof was increased to a buffer concentration of 30% (W/V). Except for these procedures, the same purification methods as in Example 1 were adopted. As a result, 134 grams of protein containing 1.65 million units of concentrated Factor XIII were obtained.

The invention has succeeded in securing the advantages that Factor XIII can be prepared from the by-product obtained in the course of manufacturing albumin and $\gamma$-globulin from human placenta without in any way disrupting the course of preparation of these substances, the heat treatment for inactivating the hepatitis virus which is useful from the viewpoint of medical treatment can be administered, and the contamination of the factor by pyrogens can be prevented by shortening the steps for its preparation. Furthermore, a high yield of the concentrated Factor XIII having a high degree of safety and stability can be prepared.

## Claim

Process for obtaining concentrated blood coagulation Factor XIII derived from human placenta by

1) extracting the placenta with a physiologically saline solution,

2) removing precipitates from the solution by centrifugation,

3) adding ammonium sulfate to the supernatant in a concentration of 20 to 30% (W/V),

4) recovering the supernatant and removing the precipitate therefrom,

5) adding ammonium sulfate to the supernatant in a concentration of 45 to 55% (W/V) and recovering the precipitate formed,

6) dissolving the precipitate in a phosphate buffer solution of a pH of 6 to 9 and containing about 0.005M of EDTA,

7) fractionating the Factor XIII containing solution for further purification,

8) heat-treating the purified Factor XIII containing solution for 9 to 11 hours at 55 to 65°C in the presence of a neutral amino acid, a monosaccharide, a sugar alcohol or a mixture of two or more thereof in a concentration of 10 to 20% (W/V),

9) contacting the resulting solution with a DEAE-cross-linked dextran which had been equilibrated to a pH of 6 to 9 thereby to adsorb blood coagulation Factor XIII,

10) eluting the blood coagulation Factor XIII,

11) subjecting the collected eluate to dialysis, sterilizing filtration and freeze drying, characterized in that the fractionation step (7) comprises the following steps

a) adding an alkylene oxide polymer or copolymer having a molecular weight of 2,000 to 20,000 selected from the group consisting of a polyethylene oxide homopolymer, a polypropylene oxide homopolymer and an ethylene oxidepropylene oxide copolymer in a concentration of 4 to 9% (W/V) to the solution,

b) removing the precipitate formed and recovering the supernatant,

c) adding the above-mentioned alkylene oxide polymer or copolymer to the supernatant in a concentration of 20 to 30% (W/V) and

d) recovering the precipitate formed and dissolving it in a phosphate buffer of a pH of 6 to 9.

## Revendication

Procédé pour l'obtention sous forme concentrée du facteur XIII de coagulation du sang dérivé du placenta humain par

1) extraction du placenta par une solution saline physiologique,

2) enlèvement des précipités de la solution par centrifugation,

3) adjonction de sulfate d'ammonium au surnageant avec une concentration de 20 à 30% (W/V),

4) récupération du surnageant et enlèvement des précipités qu'il contient,

5) adjonction de sulfate d'ammonium au surnageant dans une concentration de 45 à 55% (W/V) et récupération du précipité formé,

6) dissolution du précipité dans une solution tampon de phosphate avec un pH de 6 à 9 et contenant 0,005 M de EDTA,

7) fractionnement de la solution contenant le facteur XIII dans un but de purification,

8) traitement à la chaleur de la solution purifiée contenant le facteur XIII pendant 9 à 11 heures à 55°—65°C en présence d'un aminoacide neutre, d'un monosaccharure, d'un alcool de sucre ou d'un mélange de deux ou plus de ces corps dans une concentration de 10 à 20% (W/V),

9) mise en contact de la solution restante avec un dextran DEAE à liaisons croisées qui a été équilibré à un pH de 6 à 9, permettant l'adsorbtion du facteur XIII de coagulation du sang,

10) élution du facteur XIII de coagulation du sang,

11) soumission de l'éluate récolté à dialyse, filtration de stérilisation et lyophilisation, caractérisé en ce que l'étape de fractionnement (7) comprend les étapes suivantes:

a) adjonction à la solution d'un polymère ou copolymère d'oxyde d'alcoylidène d'un poids moléculaire de 2,000 à 20,000 choisi dans le groupe comprenant un homopolymère d'oxyde de polyéthylène, un homopolymère d'oxyde de polypropylène et un copolymère d'oxyde de propylèneoxyde d'éthylène dans une concentration de 4 à 9% (W/V),

b) enlèvement du précipité formé et récupération du surnageant,

c) adjonction au surnageant dudit polymère ou copolymère d'oxyde d'alcoylidène dans une concentration de 20 à 30% (W/V), et

d) récupération du précipité formé et dissolution dudit précipité dans un phosphate tampon avec un pH de 6 à 9.

## Patentanspruch

Verfahren zur Gewinnung von konzentriertem Blutkoagulationsfaktor XIII aus menschlicher Plazenta durch

1) Extrahieren der Plazenta mit einer physiologischen Kochsalzlösung,

2) Abtrennung der Fällungen aus der Lösung durch Zentrifugieren,

3) Zugabe von Ammoniumsulfat zum Überstand in einer Konzentration von 20 bis 20% (Gewicht/Volumen),

4) Gewinnen des Überstandes und Abtrennen der Fällungen,

5) Zugabe von Ammoniumsulfat zum Überstand in einer Konzentration von 45 bis 55% (Gewicht/Volumen) und Gewinnung der gebildeten Fällung,

6) Auflösen der Fällung in einer Phosphatpufferlösung mit einem pH-Wert von 6 bis 9, die etwa 0,005M EDTA enthält,

7) Fraktionieren der den Faktor XIII enthaltenden Lösung zur weiteren Reinigung,

8) Wärmebehandlung der den gereinigten Faktor XIII enthaltenden Lösung während 9 bis 11 Stunden bei 55 bis 65°C in Gegenwart einer neutralen Aminosäure, eines Monosaccharids, eines Zuckeralkohols oder eines Gemisches aus zwei oder mehr dieser Verbindungen in einer Konzentration von 10 bis 20% (Gewicht/Volumen),

9) Zusammenbringen der erhaltenen Lösung mit einem DEAE-vernetzten Dextran, das auf einen pH-Wert von 6 bis 9 äquilibriert worden ist, um den Blutkoagulationsfaktor XIII zu adsorbieren,

10) Eluieren des Blutkoagulationsfaktors XIII,

11) Dialyse des aufgefangenen Eluats, Sterilfiltrieren und Gefriertrocknen, dadurch gekennzeichnet, daß die Fraktionierstufe (7) folgende Stufen umfaßt

a) Zusatz eines Alkylenoxid-Polymers oder -Copolymers mit einem Molekulargewicht von 2000 bis 20 000 aus der Gruppe eines Polyäthylenoxid -Homopolymerisats, Polypropylenoxid-Homopolymerisats und eines Äthylenoxid-Propylenoxid-Copolymerisats in einer Konzentration von 4 bis 9% (Gewicht/Volumen) zur Lösung,

b) Abtrennung der gebildeten Fällung und Gewinnung des Überstandes,

c) Zusatz des vorgenannten Alkylenoxid-Polymers oder Copolymers zum Überstand in einer Konzentration von 20 bis 30% (Gewicht/Volumen) und

d) Gewinnung der gebildeten Fällung und Auflösen in einem Phosphatpuffer mit einem pH-Wert von 6 bis 9.